(19) Europäisches Patentamt
European Patent Office
Office européen des brevets

(11) EP 4 183 391 A1

(12) **EUROPEAN PATENT APPLICATION**
published in accordance with Art. 153(4) EPC

(43) Date of publication:
**24.05.2023 Bulletin 2023/21**

(21) Application number: **21846859.3**

(22) Date of filing: **19.07.2021**

(51) International Patent Classification (IPC):
***A61K 31/137*** *(2006.01)* ***A61K 31/485*** *(2006.01)*
***A61P 25/00*** *(2006.01)*

(52) Cooperative Patent Classification (CPC):
**A61K 31/137; A61K 31/485; A61P 25/00**

(86) International application number:
**PCT/CN2021/106973**

(87) International publication number:
**WO 2022/017300 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.07.2020 CN 202010698924**
**15.07.2021 CN 202110799063**

(71) Applicant: **Shenzhen Salubris Pharmaceuticals Co., Ltd.**
**Shenzhen, Guangdong 518017 (CN)**

(72) Inventors:
- **XIAO, Ying**
  **Shenzhen, Guangdong 518040 (CN)**
- **XING, Wei**
  **Shenzhen, Guangdong 518040 (CN)**
- **ZHANG, Zhixin**
  **Shenzhen, Guangdong 518040 (CN)**
- **WU, Junjun**
  **Shenzhen, Guangdong 518040 (CN)**
- **HU, Xuefeng**
  **Shenzhen, Guangdong 518040 (CN)**
- **SUN, Jingchao**
  **Shenzhen, Guangdong 518040 (CN)**

(74) Representative: **Zimmermann & Partner Patentanwälte mbB**
**Postfach 330 920**
**80069 München (DE)**

(54) **PHARMACEUTICAL COMPOSITION AND USE THEREOF**

(57) The present invention relates to the technical field of the use of a pharmaceutical composition, and in particular relates to a pharmaceutical composition and the use thereof.

EP 4 183 391 A1

## Description

### FIELD OF THE INVENTION

The present invention relates to the technical field of the use of a pharmaceutical composition, and in particular relates to a pharmaceutical composition and the use thereof.

### BACKGROUND OF THE INVENTION

Dextromethorphan (CAS: 125-71-3), the chemical name of which is (+)-3-methoxy-17-methyl-(9α, 13α, 14α)-morphinane, is mostly used clinically as its hydrobromide salt (CAS: 125-69-9). A deuterated compound of dextromethorphan can be obtained according to CN101687868A and the like in the prior arts. For example, the chemical structure of a deuterated dextromethorphan is as follows:

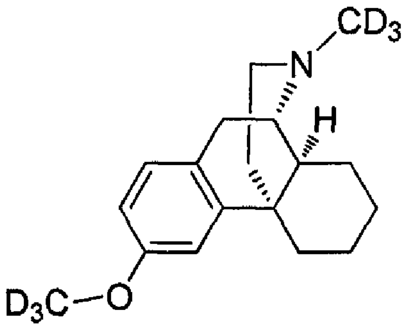

(I) .

Bupropion (CAS: 34911-55-2), the chemical name of which is 1-(3-chlorophenyl)-2-[(1,1-dimethylethyl)amino]-1-propanone, is used clinically as its hydrochloride salt (CAS: 31677-93-7). The chemical structure of bupropion is as follows:

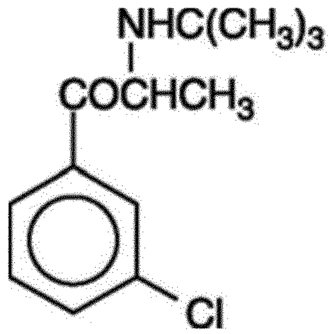

.

CN106163522A discloses a composition comprising bupropion or a related compound and dextromethorphan, and a method for use in antidepressants and other neurological diseases. Currently, the relevant medicament is in the clinical stage. There is still a wide range of unaddressed clinical needs in this area.

### SUMMARY OF THE INVENTION

In view of the clinical needs of the prior arts, the primary object of the present invention is to provide a pharmaceutical composition, wherein the pharmaceutical composition comprises a deuterated dextromethorphan or a salt thereof, and a bupropion drug or a salt thereof.

In the present invention, the bupropion drug is selected from the group consisting of bupropion, S-bupropion, R-bupropion, hydroxybupropion, erythro-hydroxybupropion and threo-hydroxybupropion, or any combination thereof.

The pharmaceutical composition of the present invention comprises a combination of the deuterated dextromethorphan or the salt thereof and the bupropion drug or the salt thereof in any ratio, in particular in a weight ratio of 1-10: 1-10, including but not limited to: 1 : 1, 2 : 1, 3 : 1, 4 : 1, 5 : 1, 6 : 1, 7 : 1, 8 : 1, 9 : 1, 10 : 1, 2 : 1, 3 : 1, 4 : 1, 5 : 1, 6 : 1, 7 : 1, 8 : 1, 9 : 1 or 10 : 1. According to a preferred embodiment of the present invention, the pharmaceutical composition is selected from the group consisting of: a combination of the deuterated dextromethorphan or the salt

thereof and bupropion or the salt thereof, a combination of the deuterated dextromethorphan or the salt thereof and S-bupropion or a salt thereof, a combination of the deuterated dextromethorphan or the salt thereof and R-bupropion or a salt thereof, a combination of the deuterated dextromethorphan or the salt thereof and hydroxybupropion or a salt thereof, a combination of the deuterated dextromethorphan or the salt thereof and the erythro-hydroxybupropion or a salt thereof, and a combination of the deuterated dextromethorphan or the salt thereof and threo-hydroxybupropion or a salt thereof.

According to a preferred embodiment of the present invention, the pharmaceutical composition comprises: the deuterated dextromethorphan or the salt thereof in terms of deuterated dextromethorphan and the bupropion or the salt thereof in terms of bupropion in a weight ratio of 1 : 1-10;

or the deuterated dextromethorphan or the salt thereof in terms of deuterated dextromethorphan and S-bupropion or the salt thereof in terms of S-bupropion in a weight ratio of 1 : 1-10;
or the deuterated dextromethorphan or the salt thereof in terms of deuterated dextromethorphan and R-bupropion or the salt thereof in terms of R-bupropion in a weight ratio of 1 : 1-10;
or the deuterated dextromethorphan or the salt thereof in terms of deuterated dextromethorphan and hydroxybupropion or the salt thereof in terms of hydroxybupropion in a weight ratio of 1 : 1-10;
or the deuterated dextromethorphan or the salt thereof in terms of deuterated dextromethorphan and erythro-hydroxybupropion or the salt thereof in terms of erythro-hydroxybupropion in a weight ratio of 1 : 1-10;
or the deuterated dextromethorphan or the salt thereof in terms of deuterated dextromethorphan and threo-hydroxybupropion or the salt thereof in terms of threo-hydroxybupropion in a weight ratio of 1 : 1-10.

According to a preferred embodiment of the present invention, the pharmaceutical composition comprises: the deuterated dextromethorphan or the salt thereof in terms of deuterated dextromethorphan and bupropion or the salt thereof in terms of bupropion in a weight ratio of 1 : 1-8;

or the deuterated dextromethorphan or the salt thereof in terms of deuterated dextromethorphan and S-bupropion or the salt thereof in terms of S-bupropion in a weight ratio of 1 : 1-8;
or the deuterated dextromethorphan or the salt thereof in terms of deuterated dextromethorphan and R-bupropion or the salt thereof in terms of R-bupropion in a weight ratio of 1 : 1-8;
or the deuterated dextromethorphan or the salt thereof in terms of deuterated dextromethorphan and hydroxybupropion or the salt thereof in terms of hydroxybupropion in a weight ratio of 1 : 1-8;
or the deuterated dextromethorphan or the salt thereof in terms of deuterated dextromethorphan and erythro-hydroxybupropion or the salt thereof in terms of erythro-hydroxybupropion in a weight ratio of 1 : 1-8;
or the deuterated dextromethorphan or the salt thereof in terms of deuterated dextromethorphan and threo-hydroxybupropion or the salt thereof in terms of threo-hydroxybupropion in a weight ratio of 1 : 1-8.

According to a preferred embodiment of the present invention, the pharmaceutical composition comprises: the deuterated dextromethorphan or the salt thereof in terms of deuterated dextromethorphan and bupropion or the salt thereof in terms of bupropion in a weight ratio of 1 : 1-6;

or the deuterated dextromethorphan or the salt thereof in terms of deuterated dextromethorphan and S-bupropion or the salt thereof in terms of S-bupropion in a weight ratio of 1 : 1-6;
or the deuterated dextromethorphan or the salt thereof in terms of deuterated dextromethorphan and R-bupropion or the salt thereof in terms of R-bupropion in a weight ratio of 1 : 1-6;
or the deuterated dextromethorphan or the salt thereof in terms of deuterated dextromethorphan and hydroxybupropion or the salt thereof in terms of hydroxybupropion in a weight ratio of 1 : 1-6;
or the deuterated dextromethorphan or the salt thereof in terms of deuterated dextromethorphan and erythro-hydroxybupropion or the salt thereof in terms of erythro-hydroxybupropion in a weight ratio of 1 : 1-6;
or the deuterated dextromethorphan or the salt thereof in terms of deuterated dextromethorphan and threo-hydroxybupropion or the salt thereof in terms of threo-hydroxybupropion in a weight ratio of 1 : 1-6.

According to a preferred embodiment of the present invention, the pharmaceutical composition comprises: the deuterated dextromethorphan or the salt thereof in terms of deuterated dextromethorphan and bupropion or the salt thereof in terms of bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.5, 1 : 3.75, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

or the deuterated dextromethorphan or the salt thereof in terms of deuterated dextromethorphan and S-bupropion

or the salt thereof in terms of S-bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

or the deuterated dextromethorphan or the salt thereof in terms of deuterated dextromethorphan and R-bupropion or the salt thereof in terms of R-bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

or the deuterated dextromethorphan or the salt thereof in terms of deuterated dextromethorphan and hydroxybupropion or the salt thereof in terms of hydroxybupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

or the deuterated dextromethorphan or the salt thereof in terms of deuterated dextromethorphan and erythro-hydroxybupropion or the salt thereof in terms of erythro-hydroxybupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

or the deuterated dextromethorphan or the salt thereof in terms of deuterated dextromethorphan and the threo-hydroxybupropion or the salt thereof in terms of threo-hydroxybupropion in a weight ratio 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10.

According to a preferred embodiment of the present invention, the pharmaceutical composition comprises the salt of the deuterated dextromethorphan and the salt of bupropion in a weight ratio of 10-18 : 50, preferably in a weight ratio of 10 : 50, 12 : 50, 15 : 50 or 18 : 50.

In an embodiment of the present invention, the pharmaceutical composition comprises 10 to 500 mg, 20 to 200 mg, 25 to 100 mg, or 30 to 80 mg of the deuterated dextromethorphan in a combination with one of bupropion, S-bupropion, R-bupropion, hydroxybupropion, erythro-hydroxybupropion or threo-hydroxybupropion in a weight ratio of 1 : 1, 1 : 1.25, 1 : 1.5,

1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10, respectively.

For example, the pharmaceutical composition comprises 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg or 80 mg of the deuterated dextromethorphan in a combination with one of bupropion, S-bupropion, R-bupropion, hydroxybupropion, erythro-hydroxybupropion or threo-hydroxybupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10, respectively.

In an embodiment of the present invention, the pharmaceutical composition comprises 10 to 500mg, 20 to 200mg, 25 to 100mg, or 30 to 80mg of the salt of the deuterated dextromethorphan. Specifically, for example, the pharmaceutical composition comprises 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg or 80 mg of the salt of the deuterated dextromethorphan.

In an embodiment of the present invention, the pharmaceutical composition comprises 10 to 500mg, 50 to 300mg, or 100 to 200mg of the salt of bupropion. Specifically, for example: the pharmaceutical composition comprises 100 mg, 101 mg, 102 mg, 103 mg, 104 mg, 105 mg, 106 mg, 107 mg, 108 mg, 109 mg, 110 mg, 111 mg, 112 mg, 113 mg, 114 mg, 115 mg, 116 mg, 117 mg, 118 mg, 119 mg, 120 mg, 121 mg, 122 mg, 123 mg, 124 mg, 125 mg, 126 mg, 127 mg, 128 mg, 129 mg, 130 mg, 132 mg, 133 mg, 134 mg, 135 mg, 136 mg, 137 mg, 138 mg, 139 mg, 140 mg, 141 mg, 142 mg, 143 mg, 144 mg, 145 mg, 146 mg, 147 mg, 148 mg, 149 mg, 150 mg, 151 mg, 152 mg, 153 mg, 154 mg, 155 mg, 156 mg, 157 mg, 158 mg, 159 mg, 160 mg, 161 mg, 162 mg, 163 mg, 164 mg, 165 mg, 166 mg, 167 mg, 168 mg, 169 mg, 170 mg, 171 mg, 172 mg, 173 mg, 174 mg, 175 mg, 176 mg, 177 mg, 178 mg, 179 mg, 180 mg, 181 mg,

182 mg, 183 mg, 18 mg, 185 mg, 186 mg, 187 mg, 188 mg, 189 mg, 190 mg, 191 mg, 192 mg, 193 mg, 194 mg, 195 mg, 196 mg, 197 mg, 198 mg, 199 mg or 200 mg of the salt of bupropion.

According to a preferred embodiment of the present invention, the pharmaceutical composition comprises a deuterated dextromethorphan represented by structural formula I:

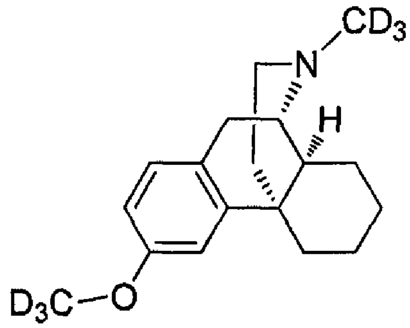

(I) .

According to a preferred embodiment of the present invention, the salt of the deuterated dextromethorphan is preferably

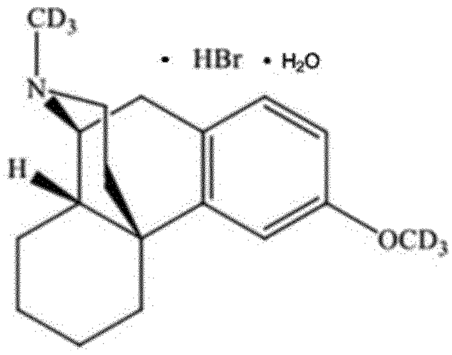

or

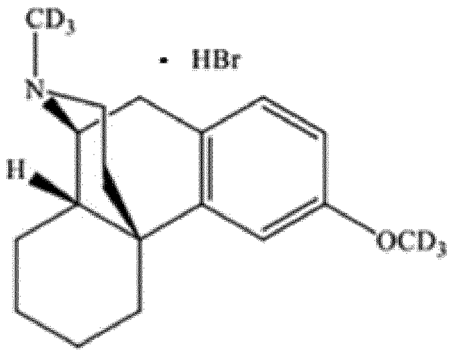

,

and the salt of bupropion is preferably

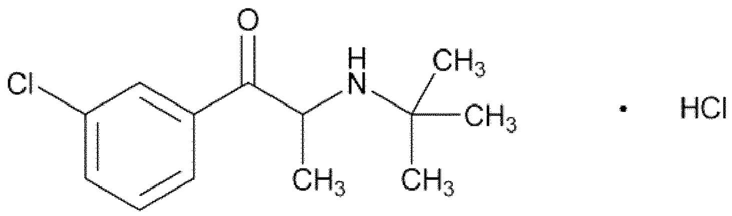

.

The structures, names, and CAS numbers of some of the compounds are as follows:

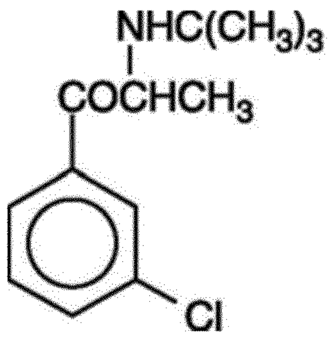

Bupropion, 34911-55-2;

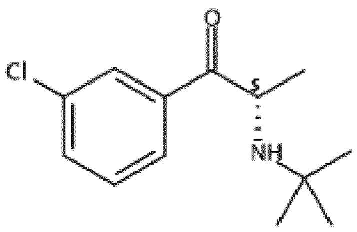

(S)-Bupropion, 324548-43-8;

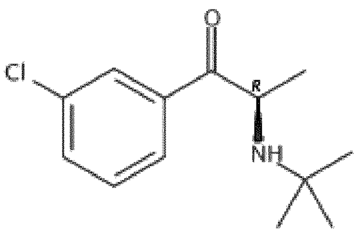

(R)-Bupropion, 437723-96-1;

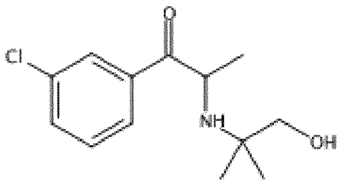

Hydroxybupropion, 92264-81-8;

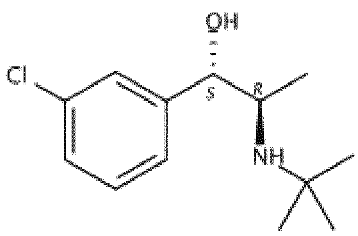

Erythro-hydroxybupropion, 99102-04-2;

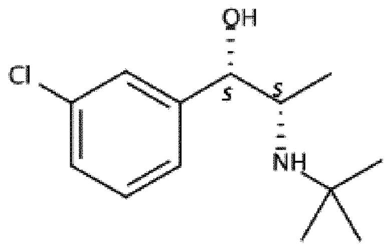

Threo-hydroxybupropion, 92264-82-9.

The active compound in the pharmaceutical compositions according to the present invention further includes any salt, solvate or hydrate thereof.

The salts of the compound of the present invention are formed from an acid with a basic group (such as an amino functional group) of the compound or from a base with an acidic group (such as a carboxyl functional group) of the compound. According to a further embodiment, the compound is a pharmaceutically acceptable acid addition salt. Generally, a variety of pharmaceutically acceptable salts can be selected.

As used in the present invention, the term "pharmaceutically acceptable" refers to those ingredients which are, within the scope of sound medical judgment, suitable for use in contact with the tissues of human and other mammals without excessive toxicity, irritation, allergic reactions, or other problem complications commensurate with a reasonable benefit/risk ratio.

The acid that is commonly used to form a pharmaceutically acceptable salt includes an inorganic acid, such as hydrogenbisulfide, hydrochloric acid, hydrobromic acid, hydroiodate acid, sulfuric acid and phosphoric acid; and an organic acid such as, p-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulphonic acid, ethanesulphonic acid, benzenesulphonic acid, lactic acid, oxalic acid, p-bromobenzenesulphonic acid, carbonic acid, succinic acid, citric acid, benzoic acid and acetic acid; and related inorganic and organic acids. Therefore, the pharmaceutically acceptable salt includes sulphate, pyrosulphate, hydrosulphate, sulphite, bisulphite, phosphate, monohydrogen phosphate, dihydrogen phosphate, metaphosphate, pyrophosphate, hydrochloride, bromate, iodate, acetate, propionate, decanoate, octanoate, acrylate, formate, isobutyrate, caprate, heptanoate, propiolate, oxalate, malonate, succinate, suberate, sebacate, fumarate, maleate, salt of butyne-1,4-dicarboxylic acid, hexyne-1,6-dicarboxylic acid, benzoate, chlorobenzoate, methylbenzoate, dinitrobenzoate, hydroxybenzoate, methoxybenzoate, phthalate, terephthalate, sulfonate, xylenesulfonate, phenylacetate, phenylpropionate, phenylbutyrate, citrate, lactate, β-hydroxybutyrate, salt of hydroxyacetic acid, maleate, tartarate, methanesulfonate, propanesulfonate, naphthalene-1-sulfonate, naphthalene-2-sulfonate, mandelate and other salts.

In one embodiment, the pharmaceutically acceptable acid addition salt includes a salt formed with an inorganic acid, such as bupropion hydrochloride, deuterated dedextromethorphan hydrobromide and the like.

As used in the present invention, the term "hydrate" means a compound further comprising stoichiometric or non-stoichiometric water bound by non-covalent intermolecular force.

As used in the present invention, the term "solvate" means a compound further comprising stoichiometric or non-stoichiometric solvent (such as water, acetone, ethanol, methanol, methylene chloride, 2-propanol, etc.) bound by non-covalent intermolecular force. "D" refers to deuterium.

The pharmaceutical composition of the present invention includes the pharmaceutical composition suitable for transoral, rectal, transnasal, topical (including trans-buccal and sublingual), vaginal or non-transintestinal (including subcutaneous, intramuscular, intravenous and intradermal) administration.

The preparation method comprises a step of binding the molecule to be administered with an ingredient, such as a vehicle, constituting one or more auxiliary ingredients. Generally, the composition is prepared by homogenizing the active ingredient in close combination with a liquid vehicle, a liposome or a fine powdered solid vehicle or both, followed by shaping the product if necessary.

In certain embodiments, the compound is administered orally. Compositions of the present invention suitable for oral administration may be provided in the forms of: individual units each containing a predetermined amount of the active ingredient (such as capsules, cachets or tablets); powders or granules; solutions or suspensions in aqueous or non-aqueous liquids; oil-in-water liquid emulsions; water-in-oil liquid emulsions; filled in liposomes; or bolus, etc. Soft gelatin capsules may be suitable for containing the suspension, which may advantageously increase the absorption rate of the compound.

In the case of tablets for oral administration, common vehicles include lactose and corn starch. Generally, lubricants such as magnesium stearate are also added. For transoral administration in capsule form, suitable diluents include lactose and dried corn starch. When administered transorally with aqueous suspensions, the active ingredient is combined with an emulsifier and a suspending agent. If necessary, certain sweeteners and/or flavourings and/or

colouring agents may be added.

Compositions suitable for non-transintestinal administration include aqueous and non-aqueous sterile injectable solutions that may contain an antioxidant, buffer, bacteriostatic agent and solute that render the formulation isotonic with the blood of the intended recipient; and aqueous and non-aqueous sterile suspensions that may contain a suspending agent and thickening agent. The formulation can be supplied in single- or multi-dose containers such as sealed ampoules and vials and can be stored in a freeze-dried (lyophilised) state of only adding sterile liquid vehicle such as water for injection immediately prior to use. Temporary injectable solutions and suspensions can be prepared from sterile powders, granules and tablets.

The injection solution may be in the form of, for example, a sterile injectable aqueous or oily suspension.

Additionally, the pharmaceutical composition of the present invention can be administered as nasal aerosols or inhalants.

The present invention further provides use of the above pharmaceutical composition in the manufacturing of a medicament for preventing and/or treating neurological diseases.

The neurological diseases include, but are not limited to, depression, major depression, refractory depression and refractory bipolar depression.

More specifically, the depression includes bipolar disorder of cyclic affective psychosis, seasonal affective disorder, mania, anxiety disorder, attention deficit disorder (ADD), attention deficit disorder with hyperactivity (ADDH), attention deficit/hyperactivity disorder (AD/HD), bipolar and manic disorder, obsessive-compulsive neurological disorder, anorexia, obesity or weight gain, narcolepsy, chronic fatigue syndrome, premenstrual syndrome, substance addiction or abuse, nicotine addiction, psychosexual dysfunction, pseudobulbar affect and emotional instability.

The beneficial effects of the present invention relative to the prior art include:

(1) Compared with the prior art, the pharmaceutical composition of the present invention has a superior metabolic effect, ensuring appropriate drug concentrations and effects for drug administration.
(2) An excellent synergistic effect can be obtained when the deuterated dextromethorphan and the bupropion in the pharmaceutical composition of the present invention is in a specific weight ratio, preferably in a weight ratio of 1:1-10, more preferably in a weight ratio of 1:1-8, particularly preferably in a weight ratio of 1:1-6.
(3) The effect of the specific ratio of the pharmaceutical composition of the present invention is superior to that of the combination of non-deuterated dextromethorphan and bupropion.
(4) The pharmaceutical composition of the present invention can be widely used in cough and various neurological conditions and diseases, and has an application effect of broad spectrum.

## DETAILED DESCRIPTION OF THE INVENTION

The present invention is further described in detail below in combination with examples, but the embodiments of the present invention are not limited to these examples.

The raw materials used in the examples of the present invention are dextromethorphan hydrobromide, deuterated dextromethorphan and bupropion hydrochloride, wherein the mass of each material used in the experiment is calculated by using free concentration, i.e. dextromethorphan hydrobromide is calculated in terms of dextromethorphan and bupropion hydrochloride is calculated in terms of bupropion.

Unless otherwise stated, the structural formulae of deuterated dextromethorphan and bupropion in the examples of the present invention are as follows, respectively:

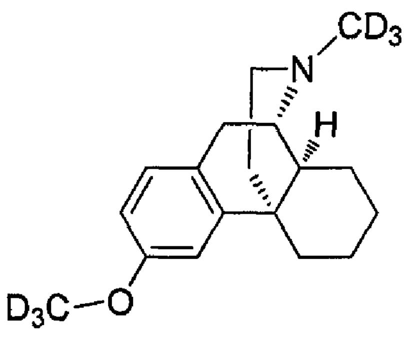
(I) ;
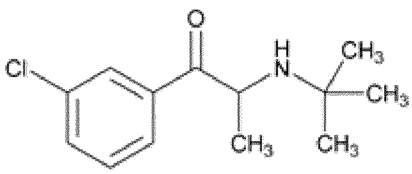
.

Unless otherwise stated, the raw material used for the deuterated dextromethorphan is deuterated dextromethorphan hydrobromide monohydrate and the raw material used for the bupropion is bupropion hydrochloride in the

examples. The structural formulae for deuterated dextromethorphan hydrobromide monohydrate and bupropion hydrochloride are as follows, respectively:

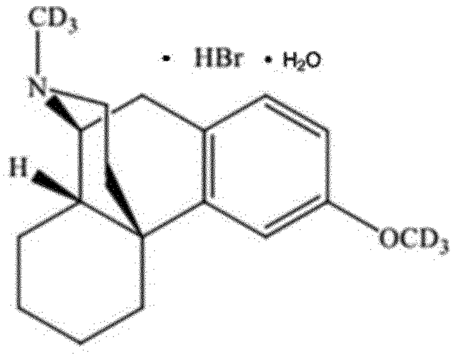

;

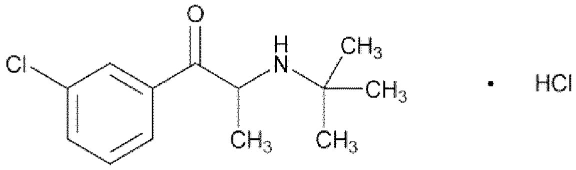

.

in which, the molecular weight of deuterated dextromethorphan hydrobromide monohydrate is calculated as 376.37, the molecular weight of deuterated dextromethorphan is calculated as 277.44, the molecular weight of bupropion hydrochloride is calculated as 276.20 and the molecular weight of bupropion is calculated as 239.74.

**Example 1. Stability assay of liver microsome**

**1.1 Experimental steps:**

1. Preparation of 0.1 M potassium phosphate buffer (pH 7.4)

Buffer A: 680.5 mg of potassium dihydrogen phosphate and 20.23 mg of EDTA were weighed and taken in a blue-capped bottle and 50 mL of purified water was added to prepare 0.1 M potassium dihydrogen phosphate buffer (13.61 mg/mL containing 1.0 mM EDTA, 0.4 mg/mL). Buffer B: 4564.4 mg dipotassium hydrogen phosphate trihydrate and 80.9 mg EDTA were weighed and taken in a blue-capped bottle and 200 mL purified water was added to prepare 0.1 M dipotassium hydrogen phosphate buffer (17.42 mg/ mL containing 1.0 mM EDTA, 0.4 mg/mL).
Buffer C: Buffer B was added to Buffer A and the pH was adjusted to 7.4 with a pH meter to obtain 0.1 M potassium phosphate buffer (containing 1.0 mM EDTA, pH 7.4).

2. Preparation of working solutions
① Stock Solution 1: Dextromethorphan hydrobromide, deuterated dextromethorphan hydrobromide monohydrate and bupropion hydrochloride powders in appropriate amount were precisely weighed, added with methanol/water (v/v=1:1) and dissolved so that the concentration of dextromethorphan is 6.0 mg/mL (free concentration), the concentration of the deuterated dextromethorphan is 6.0 mg/mL and the concentration of bupropion is 84.0 mg/mL (free concentration). 84.0 mg/mL bupropion solution was diluted with methanol/water (v/v=1:1) as described in the table below, and equal volumes of diluted bupropion solution and dextromethorphan solution or bupropion solution and deuterated dextromethorphan solution were taken and mixed to prepare a mixed stock solution 1 of dextromethorphan/deuterated dextromethorphan + bupropion in different ratios.
Preparation of mixed stock solution of dextromethorphan/deuterated dextromethorphan + bupropion

| Volume of 84.0 mg/ mLbupropion hydrochloride solution for use (μL) | Volume of methanol/water (v/v=1:1) added (μL) | Concentration of diluted bupropion (mg/mL) | Volume of diluted bupropion solution for use (μL) | Volume of 6.0 mg/ mLdextromethorphan/d euterated dextromethorphan solution for use (μL) | Weight ratio of deuterated dextromethorphan to bupropion | Weight ratio of dextromethorphan to bupropion |
|---|---|---|---|---|---|---|
| 84 | 0 | 84 | 30 | 30 | 1:14 | |
| 60 | 10 | 72 | 30 | 30 | 1:12 | |
| 60 | 24 | 60 | 30 | 30 | 1:10 | |
| 40 | 30 | 48 | 30 | 30 | 1:8 | |
| 30 | 40 | 36 | 30 | 30 | 1:6 | |
| 24 | 60 | 24 | 30 | 30 | 1:4 | 1:4 |
| 21 | 63 | 21 | 30 | 30 | 1:3.5 | 1:3.5 |
| 18 | 66 | 18 | 30 | 30 | 1:3 | 1:3 |
| 15 | 69 | 15 | 30 | 30 | 1:2.5 | 1:2.5 |
| 12 | 72 | 12 | 30 | 30 | 1:2 | 1:2 |
| 6 | 78 | 6 | 30 | 30 | 1:1 | 1:1 |
| 3 | 81 | 3 | 30 | 30 | 1:0.5 | |
| 0 | 60 | 0 | 30 | 30 | 1:0 | |

② Working solution: 60 μL of the mixed stock solution 1 obtained in step 1 was taken and diluted with 140 μL of potassium phosphate buffer to obtain a working solution (30 μM).

3. A 96-well plate was prepared by marking wells T0, T20 and T60.

4. 18.8 μL (20 mg protein/mL) of liver microsomes was added to 456.2 μL of buffer, then added with 25 μL of the working solution of dextromethorphan/deuterated dextromethorphan + bupropion or positive control obtained in step ② and mixed well with a pipette.

5. The above mixture was dispensed into the corresponding wells marked as T0, T20 and T60 in two parallel replicate wells, 30 μL each well. 50 ng/mL propranolol (internal standard IS) in acetonitrile termination solution (150 μL/well) was immediately added to well T0, followed by 15 μL NADPH solution (5 mg/mL) and mixed well.

6. Once the set time was reached, the termination solution (150 μL/well) was added to terminate the reaction, mixed well, then added with 100 μL of purified water to the wells at each time point (including T0) and mixed well again.

7. The 96-well plate was centrifuged at 4000 rpm for 5 minutes.

8. 200 μL of supernatant was transferred to a new 96-well plate for LC/MS/MS analysis.

**1.2 Data analysis:**

The peak area ratio of the analyte (dextromethorphan or deuterated dextromethorphan) / the internal standard was converted to a residual percentage (residual%) using the following equation:

$$\text{Residual \%} = \text{peak area ratio of the analyte to IS at each time point / peak area ratio of the analyte to IS at } t = 0 \times 100$$

The slope was calculated based on the residual percentage at each time point and the half-life of dextromethorphan or deuterated dextromethorphan was calculated.

**1.3 Experimental result:**

| Compound | Weight ratio | Half-life of dextromethorphan ($T_{1/2}$) (min) |
|---|---|---|
| Dextromethorphan : bupropion | 1 : 1 | 92.7 |
| | 1 : 2 | 100.8 |
| | 1 : 2.5 | 109.4 |
| | 1 : 3 | 93.8 |
| | 1 : 3.5 | 102 |
| | 1 : 4 | 106.9 |
| Compound | Weight ratio | Half-life of deuterated dextromethorphan ($T_{1/2}$) |
| Deuterated | 1 : 0 | 133.7 |

(continued)

| Compound | Weight ratio | Half-life of dextromethorphan ($T_{1/2}$) (min) |
|---|---|---|
| dextromethorphan : bupropion | 1 : 0.5 | 133.1 |
| | 1 : 1 | 158.1 |
| | 1 : 2 | 166.6 |
| | 1 : 2.5 | 183.3 |
| | 1 : 3 | 241.2 |
| | 1 : 3.5 | 186.3 |
| | 1 : 4 | 162.5 |
| | 1 : 6 | 264.8 |
| | 1 : 8 | 176.7 |
| | 1 : 10 | 168.5 |
| | 1 : 12 | 189.0 |
| | 1 : 14 | 189.3 |

### 1.4 Result of microsome assay:

The half-life of deuterated dextromethorphan and bupropion in a weight ratio of 1:1 to 1:14 were significantly longer than that of deuterated dextromethorphan as single drug (1:0), and the synergistic effect with bupropion was well demonstrated at this ratio. The half-life of deuterated dextromethorphan and bupropion in a weight ratio of 1:0.5 was comparable to that of deuterated dextromethorphan as single drug (1:0), in which the effect of bupropion was not shown and no synergistic effect was observed. In contrast, the combination of dextromethorphan and bupropion in various weight ratios between 1:1 and 1:4 had comparable effects, and the half-life of the combination of the deuterated dextromethorphan and bupropion was significantly higher than that of the combination of dextromethorphan and bupropion at the same ratio.

## Example 2 Exploitative experiment of tolerable dose in mice

### 2.1 Laboratory animal

35 male C57BL/6 mice, weighing 20-22 g, were purchased from Beijing Vital River Laboratory Animal Technology Co., Ltd.

### 2.2 Experimental drugs: deuterated dextromethorphan and bupropion hydrochloride

Administration groups (by dose): deuterated dextromethorphan/bupropion group (12 mg/kg + 24 mg/kg), deuterated dextromethorphan/bupropion group (12 mg/kg + 48 mg/kg), deuterated dextromethorphan/bupropion group (12 mg/kg + 72 mg/kg), deuterated dextromethorphan/bupropion group (12 mg/kg + 96 mg/kg), deuterated dextromethorphan/bupropion group (12 mg/kg + 120 mg/kg), deuterated dextromethorphan/bupropion group (12 mg/kg + 144 mg/kg), deuterated dextromethorphan/bupropion group (12 mg/kg + 168 mg/kg).

### 2.3 Preparation of the formulation:

A certain amount of the drugs in terms of free base was weighed and dissolved in saline and formulated into deuterated dextromethorphan/bupropion (1.2 mg/ml + 2.4 mg/ml), deuterated dextromethorphan/bupropion (1.2 mg/ml + 4.8 mg/ml), deuterated dextromethorphan/bupropion (1.2 mg/ml + 7.2 mg/ml), deuterated dextromethorphan/bupropion (1.2 mg/ml + 9.6 mg/ml), deuterated dextromethorphan/bupropion (1.2 mg/ml + 12 mg/ml), deuterated dextromethorphan/bupropion (1.2 mg/ml + 14.4 mg/ml), deuterated dextromethorphan/bupropion (1.2 mg/ml + 16.8 mg/ml), respectively.

### 2.4 Experimental method:

Male C57BL/6 mice were randomly divided into 7 groups according to body weight, 5 mice each group, namely deuterated dextromethorphan/bupropion group (12 mg/kg+24 mg/kg, 1:2), deuterated dextromethorphan/bupropion group (12 mg/kg+48 mg/kg, 1:4), deuterated dextromethorphan/bupropion group (12 mg/kg+72 mg/kg, 1:6), deuterated dextromethorphan/bupropion group (12 mg/kg+96 mg/kg, 1:8), deuterated dextromethorphan/bupropion group (12 mg/kg+120 mg/kg, 1:10), deuterated dextromethorphan/bupropion group (12 mg/kg+144 mg/kg, 1:12), and deuterated dextromethorphan/bupropion group (12 mg/kg+168 mg/kg, 1:14), respectively. Mice in each group were administered with 1 dose of the corresponding experimental drugs to be tested in a volume of 10 ml/kg. After administration, the safety in mice was observed.

### 2.5 Experimental result

There were no significant changes in the signs of mice in the deuterated dextromethorphan/bupropion (12 mg/kg + 24 mg/kg), deuterated dextromethorphan/bupropion (12 mg/kg + 48 mg/kg), deuterated dextromethorphan/bupropion (12 mg/kg + 72 mg/kg), deuterated dextromethorphan/bupropion (12 mg/kg + 96 mg/kg) and deuterated dextromethorphan/bupropion (12 mg/kg + 120 mg/kg) groups.

However, 1 animal in the deuterated dextromethorphan/bupropion (12 mg/kg + 144 mg/kg) group had tail stiffness, stiff movement, tremors and convulsions on the ground, and 4 animals in the deuterated dextromethorphan/bupropion (12 mg/kg + 168 mg/kg) group had toxic manifestations of stiffness in hind limbs, irregular stiff movement, tremors and convulsions.

### 2.6 Experimental conclusion

Mice exhibited significant neurotoxicity at doses of dextromethorphan/bupropion deuterium equal to or greater than 12 mg/kg + 144 mg/kg. Therefore, the ratio range of the tolerable dose in mice is less than or equal to 1:10.

### Example 3 Pharmacokinetic study of single oral administration of dextromethorphan or deuterated dextromethorphan + bupropion in C57BL/6 mice

### 3.1 Preparation of the solution to be administered

Dextromethorphan hydrobromide or deuterated dextromethorphan (with the structure represented by formula I) and bupropion hydrochloride were precisely weighed and dissolved in saline by vortexing to a concentration of 4 mg/mL for dextromethorphan (free concentration) or deuterated dextromethorphan and 24 mg/mL and 32 mg/mL for bupropion (free concentration), respectively. An equal volume of dextromethorphan or deuterated dextromethorphan was mixed with 24 mg/mL and 32 mg/mL of bupropion solution, respectively, to obtain a deuterated dextromethorphan + bupropion solution with a drug concentration of 2 + 12 mg/mL for dextromethorphan. The deuterated dextromethorphan or 24 mg/mL of bupropion solution was diluted 2 times with saline, respectively, to prepare a deuterated dextromethorphan solution with a concentration of 2 mg/mL.

### 3.2 Administration of drugs and blood collection in C57BL/6 mice:

27 male C57BL/6 mice of 20-22 g were randomly divided into 3 groups: dextromethorphan + bupropion (20+120 mg/kg, drug concentration 2+12 mg/mL), deuterated dextromethorphan + bupropion (20+120 mg/kg, drug concentration 2+12 mg/mL) and deuterated dextromethorphan (20 mg/kg, drug concentration 2 mg/mL), and the above drugs were administered by gavage at 10 mL/kg respectively. Mice in each group were numbered as 1-9, with 3 mice at each time point, and blood was collected from each mouse 2-3 times, at time points of 0.25, 1.5 and 7 h for mice 1-3, 0.5, 2 and 24 h for mice 4-6, and 1 and 5 h for mice 7-9. 100 μL of whole blood was collected from the retro-orbital plexus in heparinised EP tubes and then centrifuged at 10000 rpm for 2 min. The plasma was separated. The concentration of dextromethorphan or deuterated dextromethorphan was determined by LC-MS.

### 3.3 Data statistics:

The pharmacokinetic parameters of dextromethorphan or deuterated dextromethorphan were calculated by WinNonlin using the averaged blood concentrations of three animals at the same time point.

### 3.4 Experimental result:

| Dextromethorp han or deuterated dextromethorp han parameters | Unit | Deuterated dextromethorphan/bupr opion (20 + 120 mg/kg) | Dextromethorphan/ bupr opion (20 + 120 mg/kg) | Deuterated dextromethorp han (20 mg/kg) |
|---|---|---|---|---|
| Dose ratio | \ | 1 : 6 | 1 : 6 | 1 : 0 |
| Tmax | h | 0.25 | 0.25 | 0.5 |
| Cmax | ng/ml | 438 | 315 | 269 |
| AUClast | h*ng/ ml | 940 | 652 | 387 |
| $T_{1/2}$ | h | 4.26 | 7.12 | 1.06 |

### 3.5 Conclusions of the PK assay in mice:

In the deuterated dextromethorphan single drug group, the exposure was 387 h*ng/ml and the Cmax was 269 ng/mL. In drug combination of the deuterated dextromethorphan and the bupropion (ratio 1:6), the deuterated dextromethorphan exposure was 940 h*ng/ml and the Cmax was 438 ng/mL, which showed a significant increase in exposure and Cmax compared with the single drug group, and the combination of deuterated dextromethorphan and bupropion showed a good synergistic effect. The drug combination of deuterated dextromethorphan and bupropion was significantly higher in exposure and Cmax than the drug combination of dextromethorphan and bupropion in the same ratio of 1:6. The in vivo PK assay in mice demonstrated that the combination of deuterated dextromethorphan and bupropion was superior to the combination of dextromethorphan and bupropion.

## Example 4 Experimental protocol for relieving a cough in mice

### 4.1 Reagents and instruments

Ammonium Hydroxide, ACS Reagent, 28.0-30.0% $NH_3$, AI LAN (Shanghai) Chemical Technology Co., Ltd., batch no. CEC1070004. Ultrasonic Nebulizer WH-2000, Guangdong Yuehua Medical Equipment Factory Co., Ltd.

### 4.2 Laboratory animal

30 male C57BL/6 mice, weighing 20-22 g, were purchased from Beijing Vital River Laboratory Animal Technology Co., Ltd.

### 4.3 Experimental drugs:

Dextromethorphan hydrobromide, deuterated dextromethorphan and bupropion hydrochloride.

Administration groups (by dose): groups were designed as blank solvent group, dextromethorphan (free concentration)/bupropion (free concentration) group (12 mg/kg+30 mg/kg) and deuterated dextromethorphan/bupropion (free concentration) group (12 mg/kg+30 mg/kg).

### 4.4 Preparation of formulation:

A certain amount of drug in terms of free base was weighed and dissolved in saline and formulated into deuterated dextromethorphan/bupropion (1.2 mg/ml + 3 mg/ml) and dextromethorphan/bupropion (1.2 mg/ml + 3 mg/ml), respectively.

### 4.5 Experimental method:

Male C57BL/6 mice were randomly divided into 3 groups according to total coughs in the initial screening, 10 mice each group, namely the blank solvent group, the dextromethorphan/bupropion group and the deuterated dextromethorphan/bupropion group.

Mice in each group were administered with 1 dose of the corresponding experimental drugs to be tested in a volume of 10 ml/kg. After 30 min of administration, the mice were placed in an inverted 600 ml beaker filled with concentrated ammonia saturated steam and immediately timed for 10 s, and then the mice were removed and placed in another inverted 600 ml beaker to observe the coughing of the mice and recorded the total coughs within 5 min. After 60 min of administration, the above operation was repeated to evaluate and record the total coughs in the mice within 5 min.

**4.6 Statistical method:**

All experimental data are expressed as means. Statistical analysis was carried out using Graphad Prism 5 software. One-way ANOVA test was used for comparison between groups. LSD was used for comparison between groups with equal variance. Dunnett's T3 was used for comparison between groups with unequal variance. $P < 0.05$ indicated statistical significance.

**4.7 Experimental result:**

| Group | Dose | Cough inhibition rate% | |
|---|---|---|---|
| | mg/kg | 30 min | 60 min |
| Blank solvent group | 0 | 0 | |
| Dextromethorphan/bupropion | 12+30 | 41.9** | 13.1 |
| Deuterated dextromethorphan/bupropion | 12+30 | 50.2** | 42.5**# |
| Note: ***P*<0.05 vs blank solvent group, #*P*<0.05 vs dextromethorphan/bupropion group | | | |

**4.8 Experimental conclusion**

After 30 min and 60 min of administration, the cough inhibition effect in mice of deuterated dextromethorphan/bupropion was superior to that of dextromethorphan/bupropion, and deuterated dextromethorphan/bupropion was more significant than dextromethorphan/bupropion at 60 min after administration.

**Example 5 Experimental protocol for relieving a cough in mice**

**5.1 Reagents and instruments**

Ammonium Hydroxide, ACS Reagent, 28.0-30.0% $NH_3$, Al LAN (Shanghai) Chemical Technology Co., Ltd., batch no. CEC1070004. Ultrasonic Nebulizer WH-2000, Guangdong Yuehua Medical Equipment Factory Co., Ltd.

**5.2 Laboratory animal**

88 male C57BL/6J mice, weighing 20-22 g, were purchased from Beijing Vital River Laboratory Animal Technology Co., Ltd.

**5.3 Experimental drugs:**

Deuterated dextromethorphan hydrobromide monohydrate and bupropion hydrochloride.

Administration groups (by dose): The groups were designed as blank solvent group, 3 mg/kg and 12 mg/kg deuterated dextromethorphan (free concentration) groups, 30 mg/kg bupropion (free concentration) group, and deuterated dextromethorphan/bupropion groups (3/30 mg/kg, 4/30 mg/kg, 5/30 mg/kg, 6/30 mg/kg, 7.5/30 mg/kg and 9/30 mg/kg).

**5.4 Preparation of the formulation:**

A certain amount of drug in terms of free base was weighed and dissolved in saline and formulated into 0.3 mg/ml and 1.2 mg/ml deuterated dextromethorphan (free concentration) group, 3 mg/ml bupropion (free concentration)

group and deuterated dextromethorphan/bupropion group (0.3/3 mg/ml, 0.4/3 mg/ml, 0.5/3 mg/ml, 0.6/3 mg/ml, 0.75/3 mg/ml and 0.9/3 mg/ml), respectively.

**5.5 Experimental method:**

Male C57BL/6J mice were randomly divided into 8 groups, 11 mice each group, namely blank solvent group, 3 mg/kg and 12 mg/kg deuterated dextromethorphan (free concentration) groups, 30 mg/kg bupropion (free concentration) group, and deuterated dextromethorphan/bupropion groups (3/30 mg/kg, 4/30 mg/kg, 5/30 mg/kg, 6/30 mg/kg, 7.5/30 mg/kg and 9/30 mg/kg). Mice in each group were administered with 1 dose of the corresponding experimental drugs to be tested in a volume of 10 ml/kg. After 60 min of administration, the mice were placed in an inverted 600 ml beaker filled with concentrated ammonia saturated steam and immediately timed for 10 s, and then the mice were removed and placed in another inverted 600 ml beaker to observe the coughing of the mice and recorded the total coughs within 5 min.

**5.6 Statistical method:**

All experimental data are expressed as means. Statistical analysis was carried out using Graphad Prism 5 software. One-way ANOVA test was used for comparison between groups. SNK was used for comparison between groups with equal variance. Dunnett's T3 was used for comparison between groups with unequal variance. $P < 0.05$ indicated statistical significance.

**5.7 Experimental result:**

| Group | Dose | Cough inhibition rate% |
|---|---|---|
| | mg/kg | 60 min |
| Blank solvent group | 0 | 0 |
| Deuterated dextromethorphan | 3 | 11.6 |
| | 12 | 22.5 |
| Bupropion | 30 | 9.1 |
| Deuterated dextromethorphan/bupropion group | 3+30 | 39.2* |
| | 4+30 | 50.2* |
| | 5+30 | 42.3* |
| | 6+30 | 42.7* |
| | 7.5+30 | 45.4* |
| | 9+30 | 46.8* |
| Note: **P*<0.05 vs Blank solvent group | | |

**5.8 Experimental conclusion**

Combining Examples 4 and 5, after 60 min of administration, deuterated dextromethorphan/bupropion significantly increased the cough inhibition effect in mice at doses ranging from 3+30 mg/kg to 12+30 mg/kg compared with deuterated dextromethorphan or bupropion as single drug, demonstrating a synergistic effect, and deuterated dextromethorphan/bupropion was more significant than dextromethorphan/bupropion.

**Example 6 Stability assay of liver microsome**

6.1. Experimental steps

1. Preparation of 0.1 M potassium phosphate buffer (pH 7.4)

Buffer A: about 680.5 mg of potassium dihydrogen phosphate and 20.23 mg of EDTA were weighed and taken in a glass vial and added with a certain volume of purified water to prepare 0.1 M potassium dihydrogen phosphate buffer (13.61 mg/mL, containing 1.0 mM EDTA, 0.4 mg/mL).
Buffer B: about 4564.4 mg dipotassium hydrogen phosphate trihydrate and 80.9 mg EDTA were weighed and taken in a glass vial and added with a certain volume of purified water to prepare 0.1 M dipotassium hydrogen phosphate buffer (17.42 mg/ mL, containing 1.0 mM EDTA, 0.4 mg/mL).
Buffer C: Buffer B was added to Buffer A and the pH was adjusted to 7.4 with a pH meter to obtain 0.1 M potassium phosphate buffer (containing 1.0 mM EDTA, pH 7.4).

2. Preparation of working solutions

① Stock solution 1: deuterated dextromethorphan (d3), deuterated dextromethorphan (d6) and bupropion hydrochloride powder were precisely weighed, dissolved in DMSO and then formulated into mixed stock solutions of deuterated dextromethorphan (d3) + bupropion, or deuterated dextromethorphan (d6) + bupropion in different ratios according to the weight ratio of deuterated dextromethorphan : bupropion of 1:10, 1:6, 1:4, 1:3.3, 1:3 and 1:0. 5 μL of the mixed stock solution was taken and diluted with 495 μL of 50% ACN/$H_2O$ to obtain stock solution 1.
②Working solution: 60 μL of the stock solution 1 obtained in step 1 was taken and diluted with 140 μL of potassium phosphate buffer to obtain a working solution.

3. A 96-well plate was prepared by marking wells as T0 and T60.
4. 18.8 μL (20 mg protein/mL) of liver microsomes was added to 456.2 μL of buffer, then added with 25 μL of the working solution from step 2 and mixed well with a pipette.
5. The above mixture was dispensed into the corresponding wells marked as T0 and T60 in two parallel replicate wells, 30 μL each well. 50 ng/mL propranolol (internal standard IS) in acetonitrile termination solution (150 μL/well) was immediately added to well T0, followed by 15 μL NADPH solution (5 mg/mL) and mixed well.
6. 96-well plates marked as T60 well were pre-incubated for 10 min at 37°C. 15 μL of NADPH solution was added to well T60 and reacted for 60 min. The final reaction system contained approximately 0.5 mg/mL of liver microsomes and 2 mM of NADPH.
7. Once the set time was reached, the termination solution (150 μL/well) was added to terminate the reaction, mixed well, then added with 100 μL of purified water to the wells at each time point (including T0) and mixed well again.
8. The 96-well plate was centrifuged at 4000 rpm for 5 minutes.
9. 200 μL of supernatant was transferred to a new 96-well plate for LC/MS/MS analysis.

6.2. Data analysis

The peak area ratio of the analyte / the internal standard was converted to a residual percentage (residual%) by using the following equation:

$$\text{Residual } \% = \text{peak area ratio of the analyte to IS at each time point / peak area ratio of the analyte to IS at } t = 0 \times 100$$

6.3. Experimental result

| Compound | Ratio | Residual percentage of prototype after incubation for 60 min (%) |
|---|---|---|
| Deuterated | 1 : 0 | 76.0 |

(continued)

| Compound | Ratio | Residual percentage of prototype after incubation for 60 min (%) |
|---|---|---|
| dextromethorphan (d6) : bupropion | 1 : 3 | 82.8 |
| | 1 :3.3 | 94.4 |
| | 1 : 4 | 85.9 |
| | 1 : 6 | 94.6 |
| | 1 : 10 | 86.2 |
| Deuterated dextromethorphan (d3) : bupropion | 1 : 0 | 71.0 |
| | 1 : 3 | 70.2 |
| | 1 :3.3 | 73.0 |
| | 1 : 4 | 71.1 |
| | 1 : 6 | 78.3 |
| | 1 : 10 | 80.6 |

After incubation in human liver microsomes for 60 min, the prototype residual percentage of the combination of deuterated dextromethorphan (d6) and bupropion in each ratio was higher than that of the combination of deuterated dextromethorphan (d3) and bupropion.

Note that the structural formulae for deuterated dextromethorphan (d3) and deuterated dextromethorphan (d6) are as follows:

| Deuterated dextromethorphan (d3) | Deuterated dextromethorphan (d6) |
|---|---|
| 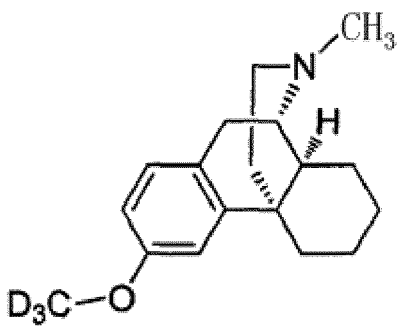 (I) | 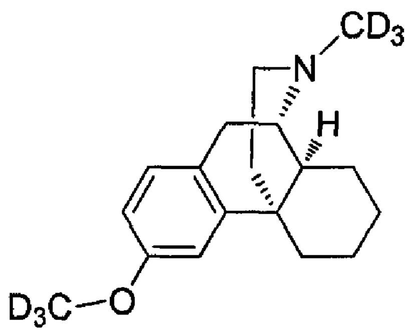 (I) |

**Example 7 Pharmacodynamic studies in a mouse tail suspension model**

**7.1 Process for experimental method**

**7.1.1 Accommodation**

110 male ICR mice were accommodated in the experimental environment for 3 days prior to the test, with 10 animals reserved for future use.

7.1.2 Grouping of animals

According to the experimental design, the mice were randomly grouped according to their body weight at one day before administration.

7.1.3 Drug administration

On the day of administration, the animals were weighed and then orally administered with saline control, deuterated dextromethorphan hydrobromide monohydrate (abbr. "deDM") and the mixed solution of deuterated dextromethorphan hydrobromide monohydrate (abbr. "deDM") / bupropion hydrochloride (abbr. "Bup") in a volume of 10 mL/kg, depending on body weight. Specific doses referred to the doses indicated in Table 7.3. The time of initiation of administration was defined as 0 min.

7.1.4 Tail suspension test

The 2 cm tail end of the mouse was attached to a horizontal metal stick so that the mouse was suspended and the immobility time period of the mouse was observed and recorded over a period of 6 min. The last 4 min of immobility time period was recorded by a timer and was used for data analysis.

### 7.2 Data collection and analysis

Excel software was used to collect data. Data were analyzed using Prism (Graphpad software, Inc.) software, in which variance between two groups was analyzed using the t-test method, and one-way ANOVA with additional Dunnett's multiple comparison test was performed among each animal group. $p<0.05$ is considered as having a significant difference.

### 7.3 Experimental results

| Group | Dose mg/kg | Ratio in terms of deuterated dextromethorphan/bupropion | Inhibition rate |
|---|---|---|---|
| Blank control | 0 | / | / |
| Bup | 50 | / | 56.00%** |
| deDM | 10 | / | 5.0% |
| deDM | 12 | / | 2.1% |
| deDM | 15 | / | 5.9% |
| deDM | 18 | / | 4.1% |
| deDM+Bup | 10+50 | 1 : 5.89 | 71.30%** |
| deDM+Bup | 12+50 | 1 : 4.91 | 89.80%** |
| deDM+Bup | 15+50 | 1 : 3.93 | 76.30%** |
| deDM+Bup | 18+50 | 1 : 3.27 | 76.30%** |

Note: comparing with blank control, **P<0.01

As seen from the above experimental result, the immobility time in the tail suspension test can be significantly reduced in the deDM+Bup drug combination group (1:3 - 1:6 in terms of deuterated dextromethorphan : bupropion) with a synergistic effect relative to different doses of deDM, and Bup.

## Example 8: Pharmacokinetic experiments in dogs

### 8.1 Experimental material

Reagents: Sodium chloride injection, EDTA-K2, acetonitrile and formic acid were commercially available.
Instrument: AB SCIEX 5500 triple quadrupole mass spectrometer.

### 8.2 Laboratory animal

18 Beagle dogs, half male and half female, weighing 6 kg-10 kg, were purchased from Jiangsu Yadong Experimental Animal Research Institute Co., Ltd.

**8.3 Experimental drugs**

Deuterated dextromethorphan hydrobromide monohydrate (deDM), dextromethorphan hydrobromide monohydrate (DM), and bupropion hydrochloride (BUP).

The doses designed for gavage administration were 14 mg/kg + 58 mg/kg for deuterated dextromethorphan hydrobromide monohydrate + bupropion hydrochloride group (deuterated dextromethorphan to bupropion in a weight ratio of 1 : 4.88), 14 mg/kg + 58 mg/kg for dextromethorphan hydrobromide monohydrate + bupropion hydrochloride group and 14 mg/kg for deuterated dextromethorphan hydrobromide monohydrate group.

**8.4 Preparation of formulation**

The test samples were precisely weighed in suitable containers, added with the target volumes of sodium chloride injection and formulated into solutions to be administered of 2.8+11.6 mg/mL for deuterated dextromethorphan hydrobromide monohydrate + bupropion hydrochloride group, 2.8+11.6 mg/mL for dextromethorphan hydrobromide monohydrate + bupropion hydrochloride group and 2.8 mg/mL for deuterated dextromethorphan hydrobromide monohydrate group.

**8.5 Blood sample collection**

After single gavage administration in dogs, about 1 mL of blood was collected with a scalp needle from a vein in the forelimb or hindlimb at 0 min (before administration), 5 min, 15 min, 30 min, 1 h, 2 h, 4 h, 8 h and 24 h in a blood collection tube containing EDTA-K2 anticoagulant and then centrifuged at 4000 rpm for 10 min to separate the plasma which was stored at ≤- 65°C for testing.

**8.6 Bioanalysis**

The concentration of deuterated dextromethorphan and dextromethorphan in the plasma of Beagle dogs was determined using a validated bioanalysis method. The standard curve concentrations of deuterated dextromethorphan in plasma were 2 ng/mL, 4 ng/mL, 20 ng/mL, 80 ng/mL, 400 ng/mL, 600 ng/mL, 1000 ng/mL and 2000 ng/mL and QC concentrations were 6 ng/mL, 100 ng/mL, 800 ng/mL and 1600 ng/mL. The standard curve concentrations of dextromethorphan in plasma were 1 ng/mL, 2 ng/mL, 10 ng/mL, 50 ng/mL, 200 ng/mL, 400 ng/mL, 600 ng/mL and 1000 ng/mL and QC concentrations were 3 ng/mL, 30 ng/mL, 500 ng/mL and 800 ng/mL.

**8.7 Data processing**

After determination and analysis of concentrations of deuterated dextromethorphan and dextromethorphan in plasma using Analyst 8.7.0 and Watson LIMS 7.4, concentration vs time curves were plotted and at least the following parameters were calculated using WinNonlin 8.1: $C_{max}$, $AUC_{0-t}$.

| Analyte | Group | Dosage (mg/kg) | $AUC_{last}$ (h*ng/ml) | | $C_{max}$ (ng/ml) | |
|---|---|---|---|---|---|---|
| | | | Male | Female | Male | Female |
| | | | Day1 | Day1 | Day1 | Day1 |
| deDM | deDM | 14 | 383 | 153 | 157 | 60 |
| deDM | deDM+BUP | 14+58 | 935 | 622 | 250 | 216 |
| DM | DM+BUP | 14+58 | 618 | 454 | 166 | 138 |

As seen from the above result, the drug combination of deuterated dextromethorphan and bupropion (weight ratio of 1:4.88) showed a significant increase in exposure and $C_{max}$ compared with single drug groups of the deuterated dextromethorphan and the drug combination of dextromethorphan and bupropion. The drug combination of deuterated dextromethorphan and bupropion demonstrated a good synergistic effect.

**Example 9 Pharmaceutical composition**

Deuterated dextromethorphan and bupropion hydrochloride (in terms of bupropion) were used to fill NO. 0 capsule in the following weight ratios, and the total weight of deuterated dextromethorphan and bupropion in each

capsule was 100 mg.

| Composition of drugs | Weight ratio (Total 100mg/capsule) |
|---|---|
| Deuterated dextromethorphan : bupropion | 1:1 |
| | 1:2 |
| | 1:2.5 |
| | 1:3 |
| | 1:3.5 |
| | 1:4 |
| | 1:6 |
| | 1:8 |
| | 1:10 |

**Example 10 Preparation method of deuterated dextromethorphan hydrobromide monohydrate**

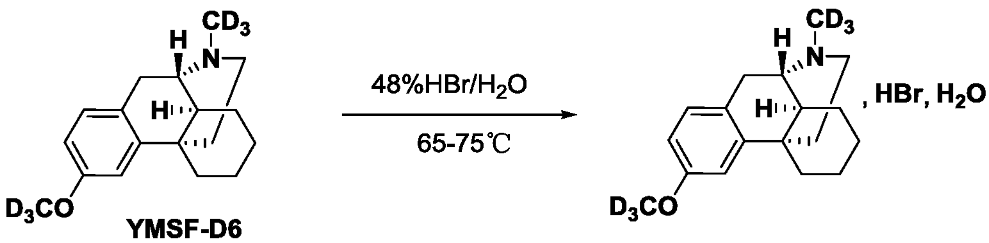


YMSF-D6

277.44 376.37

YMSF-D6 (1.0 eq) was added into 48% HBr (0.42 v/w, 1.05 eq, d=1.5) and water (5 v/w). The mixture was heated to 65-75°C, stirred for 0.5h, slowly cooled to 20-30°C, stirred for more than 0.5h, filtered, washed with water (2 v/w), and dried at 40-50°C under vacuum for 10h to obtain deuterated dextromethorphan hydrobromide monohydrate. Among others, YMSF-D6 was obtained in accordance with the prior art.

**Example 11 Preparation method of deuterated dextromethorphan hydrobromide**

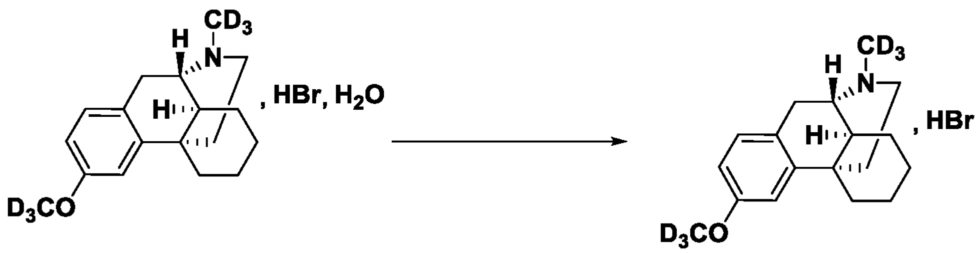

3.5 g of deuterated dextromethorphan hydrobromide monohydrate of Example 10 was weighed, added with 10 ml of methanol, and dissolved with stirring. The mixture was dropwise added to 200 ml of methyl tert-butyl ether, stirred for 5h, and subjected to suction filtration under nitrogen protection. The solid was dried under vacuum at 40°C for one day to obtain deuterated dextromethorphan hydrobromide as solid.

The above examples are preferred embodiments of the present invention, but the embodiments of the present invention are not limited by the above examples. Various changes, modifications, substitutions, combinations, simplifications made without departing from the spirit and principles of the present invention shall be equivalents and are included

within the protection scope of the present invention.

## Claims

1. A pharmaceutical composition, wherein the pharmaceutical composition comprises deuterated dextromethorphan or a salt thereof, and a bupropion drug or a salt thereof.

2. The pharmaceutical composition according to claim 1, wherein the bupropion drug is selected from the group consisting of bupropion, S-bupropion, R-bupropion, hydroxybupropion, erythro-hydroxybupropion and threo-hydroxybupropion, or any combination thereof.

3. The pharmaceutical composition according to claim 1 or 2, wherein the pharmaceutical composition comprises deuterated dextromethorphan or the salt thereof, and the bupropion drug or the salt thereof in a weight ratio of 1-10 : 1-10.

4. The pharmaceutical composition according to any one of claims 1-3, wherein the pharmaceutical composition comprises: deuterated dextromethorphan or the salt thereof in terms of deuterated dextromethorphan, and bupropion or the salt thereof in terms of bupropion in a weight ratio of 1 : 1-10, preferably in a weight ratio of 1 : 1-8, more preferably in a weight ratio of 1 : 1-6, most preferably in a weight ratio of 1 : 3 - 1 : 6;

   or deuterated dextromethorphan or the salt thereof in terms of deuterated dextromethorphan and S-bupropion or the salt thereof in terms of S-bupropion in a weight ratio of 1 : 1-10, preferably in a weight ratio of 1 : 1-8, more preferably in a weight ratio of 1 : 1-6, most preferably in a weight ratio of 1 : 3 - 1 : 6;
   or deuterated dextromethorphan or the salt thereof in terms of deuterated dextromethorphan and R-bupropion or the salt thereof in terms of R-bupropion in a weight ratio of 1 : 1-10, preferably in a weight ratio of 1 : 1-8, more preferably in a weight ratio of 1 : 1-6, most preferably in a weight ratio of 1 : 3 - 1 : 6;
   or deuterated dextromethorphan or the salt thereof in terms of deuterated dextromethorphan and hydroxybupropion or the salt thereof in terms of hydroxybupropion in a weight ratio of 1 : 1-10, preferably in a weight ratio of 1 : 1-8, more preferably in a weight ratio of 1 : 1-6, most preferably in a weight ratio of 1 : 3 - 1 : 6;
   or deuterated dextromethorphan or the salt thereof in terms of deuterated dextromethorphan and erythro-hydroxybupropion or the salt thereof in terms of erythro-hydroxybupropion in a weight ratio of 1 : 1-10, preferably in a weight ratio of 1 : 1-8, more preferably in a weight ratio of 1 : 1-6, most preferably in a weight ratio of 1 : 3 - 1 : 6;
   or deuterated dextromethorphan or the salt thereof in terms of deuterated dextromethorphan and threo-hydroxybupropion or the salt thereof in terms of threo-hydroxybupropion in a weight ratio of 1 : 1-10, preferably in a weight ratio of 1 : 1-8, more preferably in a weight ratio of 1 : 1-6, most preferably in a weight ratio of 1 : 3 - 1 : 6.

5. The pharmaceutical composition according to any one of claims 1-4, wherein the composition comprises: deuterated dextromethorphan or the salt thereof in terms of deuterated dextromethorphan and bupropion or the salt thereof in terms of bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;

   or deuterated dextromethorphan or the salt thereof in terms of deuterated dextromethorphan and S-bupropion or the salt thereof in terms of S-bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;
   or deuterated dextromethorphan or the salt thereof in terms of deuterated dextromethorphan and R-bupropion or the salt thereof in terms of R-bupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;
   or deuterated dextromethorphan or the salt thereof in terms of deuterated dextromethorphan and hydroxybupropion or the salt thereof in terms of hydroxybupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5,

1 : 7.75, 1 : 8, 1 : 8.25, 1 : 8.5, 1 : 8.75, 1 : 9, 1 : 9.25, 1 : 9.5, 1 : 9.75 or 1 : 10;
or deuterated dextromethorphan or the salt thereof in terms of deuterated dextromethorphan and erythro-hydroxybupropion or the salt thereof in terms of erythro-hydroxybupropion in a weight ratio of 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10;
or deuterated dextromethorphan or the salt thereof in terms of deuterated dextromethorphan and threo-hydroxybupropion or the salt thereof in terms of threo-hydroxybupropion in a weight ratio 1 : 1, 1 : 1.25, 1 : 1.5, 1 : 1.75, 1 : 2, 1 : 2.25, 1 : 2.5, 1 : 2.75, 1 : 3, 1 : 3.25, 1 : 3.27, 1 : 3.5, 1 : 3.75, 1 : 3.93, 1 : 4, 1 : 4.25, 1 : 4.5, 1 : 4.75, 1:4.88, 1 : 4.91, 1 : 5, 1 : 5.25, 1 : 5.5, 1 : 5.75, 1 : 5.89, 1 : 6, 1 : 6.25, 1 : 6.5, 1 : 6.75, 1 : 7, 1 : 7.25, 1 : 7.5, 1 : 7.75, 1 : 8, 1 : 8.25, 1 : 8.5, 1 : 8.75, 1 : 9, 1 : 9.25, 1 : 9.5, 1 : 9.75 or 1 : 10.

**6.** The pharmaceutical composition according to any one of claims 1-4, wherein the salt is selected from the group consisting of: sulphate, pyrosulphate, hydrosulphate, sulphite, bisulphite, phosphate, monohydrogen phosphate, dihydrogen phosphate, metaphosphate, pyrophosphate, hydrochloride, bromate, iodate, acetate, propionate, decanoate, octanoate, acrylate, formate, isobutyrate, caprate, heptanoate, propiolate, oxalate, malonate, succinate, suberate, sebacate, fumarate, maleate, salt of butyne-1,4-dicarboxylic acid, salt of hexyne-1,6-dicarboxylic acid, benzoate, chlorobenzoate, methylbenzoate, dinitrobenzoate, hydroxybenzoate, methoxybenzoate, phthalate, terephthalate, sulfonate, xylenesulfonate, phenylacetate, phenylpropionate, phenylbutyrate, citrate, lactate, β-hydroxybutyrate, salt of hydroxyacetic acid, maleate, tartarate, methanesulfonate, propanesulfonate, naphthalene-1-sulfonate, naphthalene-2-sulfonate, and mandelate.

**7.** The pharmaceutical composition according to any one of claims 1-4, wherein the pharmaceutical composition comprises the salt of deuterated dextromethorphan and the salt of bupropion in a weight ratio of 10-18 : 50, preferably in a weight ratio of 10 : 50, 12 : 50, 15 : 50 or 18 : 50.

**8.** The pharmaceutical composition according to any one of claims 1-7, wherein the pharmaceutical composition comprises deuterated dextromethorphan represented by structural formula I:

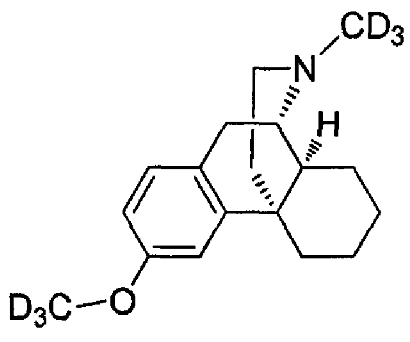

(I).

**9.** The pharmaceutical composition according to any one of claims 1-7, wherein the salt of deuterated dextromethorphan is preferably

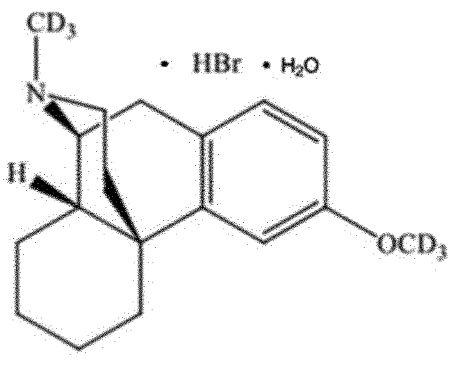

or

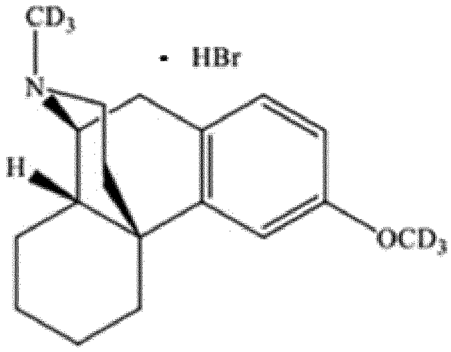

,

and the salt of bupropion is preferably

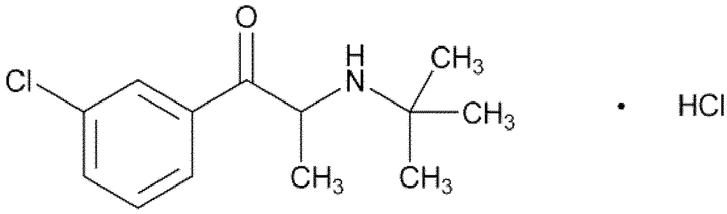

.

**10.** The pharmaceutical composition according to claim 1, wherein the pharmaceutical composition includes the pharmaceutical composition suitable for transoral, rectal, transnasal, topical (including trans-buccal and sublingual), vaginal or non-transintestinal (including subcutaneous, intramuscular, intravenous and intradermal) administration.

**11.** Use of the pharmaceutical composition according to any one of claims 1-10 in the manufacture of a medicament for preventing and/or treating cough and various neurological diseases.

**12.** The use according to claim 11, wherein the neurological diseases include but not limited to depression, major depression, refractory depression and refractory bipolar depression.

**13.** The use according to claim 12, wherein the depression includes bipolar disorder of cyclic affective psychosis, seasonal affective disorder, mania, anxiety disorder, attention deficit disorder (ADD), attention deficit disorder with hyperactivity (ADDH), attention deficit/hyperactivity disorder (AD/HD), bipolar and manic disorder, obsessive-compulsive neurological disorder, anorexia, obesity or weight gain, narcolepsy, chronic fatigue syndrome, premenstrual syndrome, substance addiction or abuse, nicotine addiction, psychosexual dysfunction, pseudobulbar affect and emotional instability.

**INTERNATIONAL SEARCH REPORT**

International application No.
**PCT/CN2021/106973**

**A. CLASSIFICATION OF SUBJECT MATTER**

A61K 31/137(2006.01)i; A61K 31/485(2006.01)i; A61P 25/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNMED; DWPI; TWABS; MOABS; TWMED; HKABS; CNABS; Pubmed; STN; ISI Web of science, CNKI: 右美沙芬, 安非他酮, 氘代右美沙芬, 口服, 咳嗽, 抑郁, Dextromethorphan, bupropion, tritiated dextromethorphan, oral, cough, depression

**C. DOCUMENTS CONSIDERED TO BE RELEVANT**

| Category* | Citation of document, with indication, where appropriate, of the relevant passages | Relevant to claim No. |
|---|---|---|
| X | CN 110279682 A (ANTECIP BIOVENTURES II L.L.C.) 27 September 2019 (2019-09-27) claims 1-24, description paragraphs [0097]-[0140], paragraphs [0208]-[0210] | 1-13 |
| X | CN 111297860 A (ANTECIP BIOVENTURES II L.L.C.) 19 June 2020 (2020-06-19) description abstract, claims 1-16, description paragraphs [0095]-[0096], [0143]-[0158] | 1-13 |
| A | CN 111343980 A (SHINKEI THERAPEUTICS L.L.C.) 26 June 2020 (2020-06-26) abstract, and claims 1-24 | 1-13 |
| A | CN 104195218 A (GUANGDONG ZHONGXI DAYI NEW DRUG DEVELOPMENT CO., LTD.) 10 December 2014 (2014-12-10) A description abstract, claims 1-9 | 1-13 |
| A | CN 101015519 A (CHONGQING PHARMACEUTICAL RESEARCH INSTITUTE CO., LTD.) 15 August 2007 (2007-08-15) abstract, and claims 1-10 | 1-13 |
| A | CN 101254249 A (ZHANG, Hui) 03 September 2008 (2008-09-03) abstract, and claims 1-10 | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2021** | **28 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/106973**

| Patent document cited in search report | Publication date (day/month/year) | Patent family member(s) | Publication date (day/month/year) |
|---|---|---|---|
| CN 110279682 A | 27 September 2019 | KR 20160070158 A | 17 June 2016 |
| | | WO 2015069809 A1 | 14 May 2015 |
| | | AU 2014346807 B2 | 22 March 2018 |
| | | CN 110251517 A | 20 September 2019 |
| | | EP 3065742 A4 | 05 April 2017 |
| | | SG 10201911808 Q A | 30 January 2020 |
| | | AU 2018203638 B2 | 18 July 2019 |
| | | MX 2016005867 A | 15 July 2016 |
| | | JP 6605485 B2 | 13 November 2019 |
| | | JP 2020023543 A | 13 February 2020 |
| | | AU 2019236614 A1 | 17 October 2019 |
| | | SG 11201603391 X A | 30 May 2016 |
| | | JP 2016535786 A | 17 November 2016 |
| | | AU 2019275593 A1 | 02 January 2020 |
| | | AU 2018203638 C1 | 24 October 2019 |
| | | SG 10201911816 X A | 30 January 2020 |
| | | CN 110327338 A | 15 October 2019 |
| | | CA 2929415 A1 | 14 May 2015 |
| | | IL 245504 D0 | 30 June 2016 |
| | | AU 2014346807 A1 | 19 May 2016 |
| | | AU 2018203638 A1 | 14 June 2018 |
| | | CN 106163522 A | 23 November 2016 |
| | | SG 10201810888 X A | 30 January 2019 |
| | | EP 3065742 A1 | 14 September 2016 |
| | | AU 2019275593 B2 | 28 January 2021 |
| | | HK 40014888 A0 | 28 August 2020 |
| | | SG 11201603391 B | 03 January 2019 |
| | | JP 6602863 B2 | 06 November 2019 |
| | | AU 2019201548 A1 | 28 March 2019 |
| | | HK 1228779 A0 | 10 November 2017 |
| | | AU 2015350559 B2 | 06 December 2018 |
| | | AU 2015350559 C | 11 July 2019 |
| | | JP 2020023527 A | 13 February 2020 |
| | | EP 3220909 B1 | 02 September 2020 |
| | | HK 40014464 A0 | 21 August 2020 |
| | | JP 2017535563 A | 30 November 2017 |
| | | MX 2017006653 A | 31 October 2017 |
| | | HK 1243323 A0 | 13 July 2018 |
| | | AU 2015350559 A1 | 08 June 2017 |
| | | US 2015126541 A1 | 07 May 2015 |
| | | SG 11201704066 A1 | 29 June 2017 |
| | | KR 101931896 B1 | 21 December 2018 |
| | | KR 20180136003 A | 21 December 2018 |
| | | EP 3220909 A1 | 27 September 2017 |
| | | HK 40015601 A0 | 04 September 2020 |
| | | KR 20170088926 A | 02 August 2017 |
| | | US 9198905 B2 | 01 December 2015 |
| | | EP 3220909 A4 | 11 July 2018 |
| | | AU 2019201548 B2 | 26 November 2020 |
| | | SG 11201704066 B | 20 July 2020 |

Form PCT/ISA/210 (patent family annex) (January 2015)

**INTERNATIONAL SEARCH REPORT**
**Information on patent family members**

International application No.
**PCT/CN2021/106973**

| Patent document cited in search report | Publication date (day/month/year) | Patent family member(s) | Publication date (day/month/year) |
|---|---|---|---|
| | | EP 3763372 A1 | 31 January 2021 |
| | | AU 2021200981 A1 | 11 March 2021 |
| | | JP 6842129 B2 | 17 March 2021 |
| CN 111297860 A | 19 June 2020 | JP 2017535563 A | 30 November 2017 |
| | | KR 101931896 B1 | 21 December 2018 |
| | | SI 3220909 T1 | 29 January 2021 |
| | | MX 2017006653 A | 31 October 2017 |
| | | HK 1243323 A1 | 13 July 2018 |
| | | HR P20201855 T1 | 02 April 2021 |
| | | CA 2968371 A1 | 26 May 2016 |
| | | RS 61113 B1 | 31 December 2020 |
| | | KR 20180136003 A | 21 December 2018 |
| | | JP 2020023527 A | 13 February 2020 |
| | | EP 3220909 B1 | 02 September 2020 |
| | | DK 3220909 T3 | 23 November 2020 |
| | | AU 2019201548 B2 | 26 November 2020 |
| | | SG 10201911873 X A | 27 February 2020 |
| | | IL 252417 A | 25 March 2021 |
| | | SG 11201704066V A | 29 June 2017 |
| | | AU 2015350559 B2 | 06 December 2018 |
| | | KR 20170088926 A | 02 August 2017 |
| | | AU 2021200981 A1 | 11 March 2021 |
| | | WO 2016081027 A1 | 26 May 2016 |
| | | EP 3220909 A4 | 11 July 2018 |
| | | JP 2021066744 A | 30 April 2021 |
| | | AU 2019201548 A1 | 28 March 2019 |
| | | CA 2968371 C | 18 August 2020 |
| | | LT 3220909 T | 25 January 2021 |
| | | IL 280905 D0 | 29 April 2021 |
| | | CN 107205998 A | 26 September 2017 |
| | | AU 2015350559 A1 | 08 June 2017 |
| | | JP 6842129 B2 | 17 March 2021 |
| | | EP 3763372 A1 | 13 January 2021 |
| | | IL 252417 D0 | 31 July 2017 |
| | | CA 3082645 A1 | 26 May 2016 |
| | | EP 3220909 A1 | 27 September 2017 |
| | | JP 6602863 B2 | 06 November 2019 |
| | | AU 2015350559 C1 | 11 July 2019 |
| | | PT 3220909 T | 07 December 2020 |
| | | US 9486450 B2 | 08 November 2016 |
| | | US 2015150830 A1 | 04 June 2015 |
| CN 111343980 A | 26 June 2020 | JP 2020536953 A | 17 December 2020 |
| | | EP 3691641 A1 | 12 August 2020 |
| | | CA 3078369 A1 | 11 April 2019 |
| | | KR 20200062254 A | 03 June 2020 |
| | | US 2020323788 A1 | 15 October 2020 |
| | | AU 2018345723 A8 | 23 April 2020 |
| | | EP 3691641 A4 | 07 April 2021 |
| | | BR 112020006723 A2 | 06 October 2020 |
| | | IL 273735 D0 | 31 May 2020 |

Form PCT/ISA/210 (patent family annex) (January 2015)

**INTERNATIONAL SEARCH REPORT**
**Information on patent family members**

International application No.
**PCT/CN2021/106973**

| Patent document cited in search report | | | Publication date (day/month/year) | Patent family member(s) | | | Publication date (day/month/year) |
|---|---|---|---|---|---|---|---|
| | | | | WO | 2019070864 | A1 | 11 April 2019 |
| | | | | TW | 201922232 | A | 16 June 2019 |
| | | | | AU | 2018345723 | A1 | 16 April 2020 |
| | | | | IN | 202047018165 | A | 12 June 2020 |
| CN | 104195218 | A | 10 December 2014 | | None | | |
| CN | 101015519 | A | 15 August 2007 | CN | 101015519 | B | 21 March 2012 |
| CN | 101254249 | A | 03 September 2008 | CN | 101254249 | B | 12 January 2011 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101687868 A
- CN 106163522 A


### Non-patent literature cited in the description


- *CHEMICAL ABSTRACTS,* 125-71-3
- *CHEMICAL ABSTRACTS,* 125-69-9
- *CHEMICAL ABSTRACTS,* 34911-55-2
- *CHEMICAL ABSTRACTS,* 31677-93-7